(19) Europäisches Patentamt
European Patent Office
Office européen des brevets

(11) EP 1 763 330 B1

(12) **FASCICULE DE BREVET EUROPEEN**

(45) Date de publication et mention de la délivrance du brevet:
**01.03.2017 Bulletin 2017/09**

(21) Numéro de dépôt: **05753741.7**

(22) Date de dépôt: **08.04.2005**

(51) Int Cl.:
***A61K 8/25*** *(2006.01)* ***A61K 8/29*** *(2006.01)*
***A61K 8/44*** *(2006.01)* ***A61K 8/19*** *(2006.01)*
***A61K 8/02*** *(2006.01)* ***A61Q 1/06*** *(2006.01)*
***A61Q 3/02*** *(2006.01)*

(86) Numéro de dépôt international:
**PCT/FR2005/000869**

(87) Numéro de publication internationale:
**WO 2005/102251 (03.11.2005 Gazette 2005/44)**

(54) **COMPOSITION DESTINÉE À LA PEAU, AUX LEVRES OU AUX ONGLES**
ZUSAMMENSETZUNG ZUR VERWENDUNG AUF HAUT, LIPPEN ODER NÄGELN
COMPOSITION FOR USE ON THE SKIN, LIPS OR NAILS.

(84) Etats contractants désignés:
**AT BE BG CH CY CZ DE DK EE ES FI FR GB GR HU IE IS IT LI LT LU MC NL PL PT RO SE SI SK TR**

(30) Priorité: **08.04.2004 FR 0450712**
**08.04.2004 FR 0450713**
**08.04.2004 FR 0450714**
**08.04.2004 FR 0450715**

(43) Date de publication de la demande:
**21.03.2007 Bulletin 2007/12**

(73) Titulaire: **L'OREAL**
**75008 Paris (FR)**

(72) Inventeurs:
- **BLIN, Xavier**
**F-75015 Paris (FR)**
- **THEVENET, Ludovic**
**92340 Bourg La Reine (FR)**
- **DUMOUSSEAUX, C.,**
**Nihon L'Oreal KSP R & D**
**Kawasaki-shi,**
**213-0012 Kanagawa-ken (JP)**

(74) Mandataire: **Nony**
**11 rue Saint-Georges**
**75009 Paris (FR)**

(56) Documents cités:
**EP-A- 1 184 426** **EP-A- 1 217 046**
**EP-A- 1 382 323** **FR-A- 2 594 130**
**US-A- 5 356 617** **US-A1- 2002 064 509**
**US-B1- 6 428 773**

Remarques:
Le dossier contient des informations techniques présentées postérieurement au dépôt de la demande et ne figurant pas dans le présent fascicule.

EP 1 763 330 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne les compositions destinées à être appliquées sur la peau, y compris les muqueuses, notamment les lèvres, et les phanères, notamment les ongles, cils, sourcils et cheveux.

L'invention concerne plus particulièrement mais non exclusivement les rouges à lèvres.

L'une des propriétés importantes de certaines compositions cosmétiques et notamment des rouges à lèvres est la couleur.

Généralement, celle-ci est obtenue par l'utilisation de pigments, ce qui se traduit par des sélections qui permettront par association d'obtenir la couleur finale recherchée.

Cependant, ces associations ne permettent pas d'obtenir une infinité de couleurs et n'autorisent qu'une zone colorielle déterminée par leur nature.

De plus, ces pigments, lorsqu'introduits dans un milieu, vont physiquement et optiquement interagir avec celui-ci, ce qui peut entraîner une modification de leur couleur.

Ce changement de couleur est entièrement conditionné par la nature du pigment et celle du milieu, ce qui rend difficile de prédire l'altération de la couleur du pigment.

Il existe un besoin pour bénéficier de pigments facilitant l'obtention de nouvelles couleurs.

Il existe également un besoin pour bénéficier de pigments dont la couleur n'est que peu altérée par la nature du milieu environnant.

Il existe encore un besoin pour obtenir des compositions présentant une variabilité relativement faible de la couleur avant et après l'application.

Enfin, pour certaines applications telles que par exemple le maquillage des lèvres, il existe un besoin pour disposer de compositions brillantes malgré une teneur en particules relativement élevée.

Le brevet US 5 356 617 décrit des matières pouvant comporter des sphères organiques ou inorganiques de taille comprise de préférence entre 1 et 100 µm.

La demande de brevet français FR 2 594 130 divulgue une poudre composite et un exemple de rouge à lèvres comportant des particules de résine de méthacrylate de méthyle, de dimension moyenne 10 µm, enrobées de matière colorante.

Le brevet US 6 428 773 divulgue une composition cosmétique comportant un pigment interférentiel de taille comprise entre 1 et 200 µm ayant une structure multicouche particulière, mélangé éventuellement à un pigment interférentiel conventionnel.

Les demandes de brevet européen EP 1 217 046 et EP 1 184 426 divulguent des peintures ou résines et non des compositions destinées à être appliquées sur la peau, les lèvres ou les phanères.

Selon l'un de ses aspects parmi d'autres, l'invention a pour objet une composition destinée à être appliquée sur la peau, les lèvres et/ou les phanères, comportant dans un milieu physiologiquement acceptable, au moins un pigment composite comportant un noyau inorganique et au moins un enrobage au moins partiel d'au moins une matière colorante organique.

Plus particulièrement la présente invention vise une composition cosmétique comportant dans un milieu physiologiquement acceptable, de 0,1 à 85 % en poids d'huile par rapport à son poids total et au moins un pigment composite comportant un noyau inorganique comportant un matériau choisi parmi le dioxyde de titane et la silice et au moins un enrobage au moins partiel d'au moins une matière colorante organique, la taille moyenne du noyau inorganique étant comprise entre 5 nm et 100 nm, la variation ΔE de la couleur de la composition avant et après application étant inférieure ou égale à 20, mieux inférieure ou égale à 15, voire 10, ledit paramètre ΔE étant déterminé selon le protocole décrit dans la description et calculé selon :

$$\Delta E = [(a^*_{masse} - a^*_{application})^2 + (b^*_{masse} - b^*_{application})^2 + (L^*_{masse} - L^*_{application})^2]^{1/2}$$

dans laquelle $L^*_{masse}$, $a^*_{masse}$ et $b^*_{masse}$ désignent les valeurs L*, a* et b* de la composition dans la masse et $L^*_{application}$, $a^*_{application}$ et $b^*_{application}$ désignent les valeurs L*, a* et b* de la composition après application dans l'espace CIE L*a*b*.

La saturation $C^*_{masse}$ de la composition est de préférence supérieure à 25. Le paramètre Δa*b* du pigment composite est de préférence supérieur ou égal à 5, mieux supérieur ou égal à 10, voire 15 ou 20.

La taille moyenne du noyau inorganique est avantageusement comprise entre 10 et 75 nm, mieux encore entre 10 et 50 nm, notamment strictement entre 10 et 50 nm, par exemple de 20 à 30 nm environ.

### Protocole de mesure du paramètre $\Delta a^*b^*_{pigment}$

La matière colorante organique pure est compactée dans une coupelle rectangulaire de dimensions 2 x 1,5

cm et une profondeur de 3 mm, en appliquant une pression de 100 bars.

Les valeurs a* et b* de la matière colorante organique compactée sont mesurées avec un spectrophotomètre Minolta 3700d, en mode spéculaire exclu ouverture moyenne et illuminant D65.

Les valeurs a* et b* du pigment composite sont également mesurées avec la même coupelle, après compression sous une pression de 100 bars, en utilisant le même spectrophotomètre.

Protocole de mesure de la saturation $C^*_{masse}$ de la composition dans la masse

Les valeurs $a^*_{masse}$ et $b^*_{masse}$ de la composition sont mesurées en utilisant un spectromètre Murakami CMS-35 FS avec fibre optique, sous illuminant D65, ouverture 3 mm et un angle de 10°. La fibre optique est mise en contact avec la composition.

Les valeurs $a^*_{masse}$ et $b^*_{masse}$ dans l'espace colorimétrique CIE L*a*b* sont mesurées six fois et moyennées.

Lorsque le produit est un stick, la couleur peut être mesurée directement sur le stick. Sinon, les mesures de $a^*_{masse}$ et $b^*_{masse}$ peuvent être effectuées sur une épaisseur de composition d'au moins 3 mm d'épaisseur.

Lorsque la composition est une poudre, celle-ci est compactée dans une coupelle rectangulaire de dimensions 1,5 x 2 cm et de profondeur 3 mm, sous une pression de 100 bars.

Avantageusement, la saturation C* de la composition est comprise entre environ 30 et environ 100.

La saturation C* de la composition, dans des exemples de mise en oeuvre de l'invention, peut être supérieure ou égale à 25, 30 ou 40.

La présente invention rend possible, selon cet aspect, d'obtenir de nouvelles teintes avec des matières colorantes organiques connues, certains pigments organiques par exemple présentant une couleur différente lorsqu'ils couvrent au moins partiellement un noyau inorganique.

L'invention permet ainsi dans certains exemples de mise en oeuvre d'obtenir de nouvelles teintes pour lesquelles il n'existe aucun pigment cosmétique connu, sans avoir à mélanger des pigments de différentes couleurs.

Selon un autre de ses aspects, indépendamment ou en combinaison avec ce qui précède, l'invention a encore pour objet une composition destinée à être appliquée sur la peau ou les lèvres et/ou les phanères, comportant des particules d'au moins un pigment composite, ces particules comportant :

- un noyau inorganique,
- au moins un enrobage au moins partiel d'au moins une matière colorante organique.

Celle-ci est de préférence dans une quantité suffisante pour que la saturation C* de la composition soit supérieure ou égale à 25.

Protocole pour mesurer ΔE

Couleur de la composition dans la masse

Les valeurs $L^*_{masse}$, $a^*_{masse}$ et $b^*_{masse}$ de la composition dans la masse sont mesurées comme décrit précédemment, en utilisant le spectrocolorimètre Murakami CMS-35 FS, sous illuminant D65, ouverture 3 mm et un angle de 10°.

Les valeurs L*, a* et b* sont mesurées six fois et moyennées.

Lorsque le produit est un stick, les valeurs $L^*_{masse}$, $a^*_{masse}$ et $b^*_{masse}$ peuvent être mesurées sur le stick, autrement elles le sont sur une épaisseur d'au moins 3 mm de composition.

Couleur de la composition après application

La composition est appliquée manuellement pour former une couche de 1 $mg/cm^2$ sur un substrat de Bio Skin® ayant pour coordonnées colorimétriques L*=69, a*=11.5 et b*=19.7.

Le substrat de Bio Skin® présente une épaisseur de 5 mm, a une surface lisse et est commercialisé par la société japonaise BEAULAX sous la référence Bio Skin n° 10, format A4.

Les valeurs $L^*_{application}$ $a^*_{application}$ et $b^*_{application}$ sont mesurées 10 fois et moyennées.

La différence ΔE est donnée par :

$$\Delta E = [(a^*_{masse} - a^*_{application})^2 + (b^*_{masse} - b^*_{application})^2 + (L^*_{masse} - L^*_{application})^2]^{1/2}.$$

La saturation de la composition après application, donnée par $C^* = [a^{*2}_{application} + b^{*2}_{application}]$ peut être su-

périeure ou égale à 30, par exemple supérieure ou égale à 40.

L'invention permet, selon cet aspect, d'obtenir des compositions ayant une faible variabilité de la couleur avant et après application.

L'invention a encore pour objet, selon un autre de ses aspects, une composition cosmétique brillante comportant au moins un pigment composite, ce pigment composite comportant des particules comportant :

- un noyau inorganique,
- au moins une matière colorante organique recouvrant au moins partiellement le noyau inorganique.

Selon cet aspect, l'invention concerne plus particulièrement mais non exclusivement les compositions liquides, par exemple les rouges à lèvres liquides.

Ainsi la composition peut se présenter sous forme liquide, pâteuse ou gélifiée.

La composition présente de préférence une brillance moyenne T0h supérieure ou égale à 30.

L'invention selon cet aspect peut s'appliquer également aux compositions solides, par exemple sous forme de stick.

Protocole de mesure de la brillance moyenne T0h :

Par « brillance moyenne », on désigne la brillance telle qu'elle peut être mesurée à l'aide d'un brillancemètre, de manière conventionnelle, par la méthode suivante.

Sur une carte de contraste de marque BYK Gardner et de référence Prüfkarten, Art. 2853, préalablement fixée sur une plaque de verre 1 mm, on étale une couche de 25 $\mu$m d'épaisseur de la composition à l'aide d'un étaleur automatique (Bar coater, Sheen). La couche recouvre au moins le fond noir de la carte. Lorsque la composition est solide, on la fait fondre si nécessaire sur la carte après l'avoir étalée afin qu'elle recouvre le fond noir. Dès que la composition est étalée, on procède à la mesure de la brillance, dite brillance moyenne T0h, à 60° sur le fond noir à l'aide d'un brillancemètre de marque BYK GARDNER et de référence microTRI-GLOSS. On prépare ainsi quatre cartes de contraste pour mesurer la brillance moyenne de la composition, et on procède à la moyenne des quatre mesures. Pour que la mesure soit correcte, l'écart type doit être inférieur ou égal à 3%.

On peut laisser ensuite la carte de contraste 5 heures sur une plaque thermostatée à une température de 30°C. Au bout de 5 heures, on retire la carte de contraste de la plaque thermostatée pour qu'elle revienne à la température de la pièce puis on procède à nouveau à la mesure de la brillance moyenne, dite brillance moyenne T5h, comme précédemment.

De préférence, lorsque la brillance est recherchée, la brillance moyenne de la composition T0h est supérieure ou égale à 45, mieux encore, supérieure ou égale à 50, mieux encore, supérieure ou égale à 60, mieux encore, supérieure ou égale à 65, mieux encore, supérieure ou égale à 70, mieux encore, supérieure ou égale à 75.

De préférence encore, la brillance moyenne de la composition T5h, une fois étalée sur un support, mesurée à 60° est supérieure ou égale à 35, mieux encore, supérieure ou égale à 40, mieux encore, supérieure ou égale à 45, mieux encore, supérieure ou égale à 50, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75, sur 100.

Indépendamment ou en combinaison avec les différents aspects de l'invention mentionnés ci-dessus, la teneur en particules de la composition peut être, dans certains exemples de mise en oeuvre de l'invention, notamment dans le cas de compositions pour lesquelles on recherche de la brillance et/ou de compositions comportant une phase grasse, supérieure ou égale à 5 % relativement au poids total de la composition, par exemple supérieure ou égale à 7,5 %, voire 10 %, 15 %, 20 % ou plus, par exemple 25 % ou 30 %.

L'utilisation de pigments composites selon l'invention peut permettre d'obtenir une brillance relativement élevée malgré une teneur relative en particules importante.

La teneur relative en particules est déterminée par le protocole de mesure suivant.

Protocole de mesure de la teneur relative Q en particules :

On utilise un Soxhlet comportant une cartouche et muni d'un ballon, d'un chauffe-ballon et d'un réfrigérant à boules.

On commence par régénérer la cartouche du Soxhlet en faisant bouillir environ 80 ml de toluène dans le ballon de manière à ce que les cycles durent environ une demi-heure. On laisse refroidir et on fait sécher la cartouche à l'étuve une nuit puis au dessicateur.

On utile une membrane de PTFE dont on connaît la masse $T_1$, que l'on plie en cône et que l'on insère dans la cartouche. On pèse précisément 0,75 g (m) de produit dans la membrane de PTFE et l'on replie la membrane dans

la cartouche de telle sorte qu'elle soit bien fermée.

On met la cartouche dans le Soxhlet après y avoir introduit un petit vial percé, servant à maintenir le haut de la cartouche un peu au-dessus du niveau du coude du Soxhlet pour éviter que le niveau du toluène ne soit supérieur au haut de la cartouche et que le toluène n'entraîne le produit.

On ajoute environ 80 ml de toluène dans le ballon. On met en route la réfrigération et on chauffe le ballon de manière à ce que le toluène soit porté à ébullition (point d'ébullition 110,6 °C) à reflux pendant quatre heures. Les vapeurs de toluène doivent se condenser à la première boule de réfrigérant et cette condensation ne doit pas être trop rapide. On laisse refroidir puis on éteint la réfrigération.

On fait sécher la cartouche à l'étuve pendant deux jours et on la place au dessicateur durant un minimum de deux heures, puis on pèse ($T_2$) la cartouche sèche contenant les insolubles (charges, pigments) immédiatement à la sortie du dessicateur. On effectue au moins deux prises d'essai de l'échantillon afin d'obtenir deux résultats concordants. La proportion de la quantité d'insolubles, c'est-à-dire qui n'est pas soluble par le toluène à chaud, qui reste dans la cartouche est donnée par $Q = ((T_2-T_1)/m) \times 100$.

Lorsque la composition est un solide, par exemple un stick de rouge à lèvres, la composition présente par exemple une dureté supérieure à 100 g, voire 110 g, voire 120 g ou plus, par exemple 130 g ou 140 g. La présence des pigments composites peut permettre d'obtenir une dureté plus élevée. Les propriétés à l'application peuvent s'en trouver changées.

<u>Protocole de mesure de la dureté :</u>

Pour déterminer la dureté de la composition solide, on prépare un stick de ladite composition ayant une section circulaire de 12,7 mm de diamètre. Le stick est coulé 24 heures avant de faire la mesure et il est conservé à une température de 20 °C.

La dureté peut être mesurée par la méthode dite « du fil à couper le beurre », qui consiste à couper transversalement le stick à l'aide d'un fil rigide de diamètre 250 µm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La dureté est exprimée en grammes.

Quels que soient les aspects de l'invention considérés, le pigment composite peut comporter ou non un liant.

De préférence, le pigment composite comporte un liant assurant la tenue de la matière colorante organique sur le noyau minéral.

Ce liant est avantageusement un liant organique, par exemple un polymère de silicone.

La teneur en pigment composite dans la composition peut être comprise entre environ 0,05 % et environ 10 % en poids, de préférence entre environ 0,1 % et 8 % en poids, mieux entre environ 0,1 % et environ 5 % en poids, par rapport au poids total de la composition, par exemple entre 0,1 % environ et 3 % environ ou entre 0,5 % environ et 3 % environ.

La quantité de pigment composite pourra dépendre des propriétés de couvrance et de saturation recherchées, notamment.

Une teinte appropriée peut être obtenue de diverses façons, par exemple par le mélange de pigments composites selon l'invention, ces pigments ayant des couleurs différentes et/ou par la présence de plusieurs matières colorantes organiques dans l'enrobage des noyaux du ou des pigments composites, ces matières colorantes organiques étant par exemple mélangées ou présentes dans des strates respectives de l'enrobage.

Par « un enrobage au moins partiel », on entend au sens de la présente invention, un enrobage de tout ou partie du noyau inorganique.

Par « milieu physiologiquement acceptable », on désigne un milieu non toxique et susceptible d'être appliqué sur la peau, les lèvres ou les phanères d'êtres humains notamment un milieu cosmétique. Le milieu physiologiquement acceptable sera adapté à la nature du support sur lequel doit être appliquée la composition, ainsi qu'à la forme sous laquelle la composition est destinée à être conditionnée, notamment solide ou fluide à température ambiante et sous pression atmosphérique.

Par « composition cosmétique », on désigne une composition telle que définie dans la Directive 93/35/CEE du Conseil du 14 juin 1993.

La composition peut, le cas échéant, présenter un pouvoir couvrant compris strictement entre 1 et 25 ou entre 25 et 100, selon que l'on cherche une faible couvrance ou une forte couvrance.

La couvrance de la composition est par exemple supérieure à 60, voire à 70, ou 80, ou 90.

La couvrance peut dépendre de la quantité de pigment composite et autres pigments ou charges éventuelles.

Mesure du pouvoir couvrant

Dans le cas d'un stick, la formulation est préalablement malaxée de façon à obtenir une pâte visqueuse.
Dans le cas d'une poudre, 50 parts en poids de la poudre sont malaxées avec 50 parts en poids de diméthicone (DC 200 Fluid 5CST de DOW CORNING) de façon à obtenir une pâte visqueuse.
La formulation est ensuite étalée avec une épaisseur de 30 $\mu$m sur une carte de contraste Erichsen, type 24/5, présentant un fond noir et un fond blanc, et les coordonnées trichromatiques (X, Y, Z) sont mesurées à l'aide d'un colorimètre CR-300.
Des étalements similaires sont réalisés sur deux autres cartes de contraste et trois mesures sont effectuées sur chaque carte. La moyenne correspondant à ces neuf mesures est ensuite calculée.
Le pouvoir couvrant est égal à 100 x Yn/Yb où Yn est la valeur moyenne de Y sur fond noir et Yb est la valeur moyenne de Y sur fond blanc. Un pouvoir couvrant de 100 correspond à une formulation complètement opaque.
La saturation C* du pigment composite est par exemple supérieure ou égale à 30, voire 35, 40 ou 45.

Protocole de mesure de la saturation du pigment composite :

Les valeurs a* et b* dans l'espace CIE L*a*b* du pigment composite sont mesurées comme suit :

Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3600d, en mode spéculaire exclu, sous illuminant D65 et ouverture moyenne. La saturation est donnée par $C^* = (a^{*2} + b^{*2})^{1/2}$.

## *PIGMENT COMPOSITE*

### *Structure*

Un pigment composite selon l'invention peut être composé notamment de particules comportant :

- un noyau inorganique,
- au moins un enrobage au moins partiel d'au moins une matière colorante organique.

Au moins un liant peut avantageusement contribuer à la fixation de la matière colorante organique sur le noyau inorganique. Ce liant peut avantageusement agir sans la formation de liaisons covalentes.
Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.
Un pigment composite selon l'invention peut présenter par exemple une surface spécifique comprise entre 1 et 1000 $m^2/g$, notamment entre 10 et 600 $m^2/g$ environ, et en particulier entre 20 et 400 $m^2/g$ environ. La surface spécifique est la valeur mesurée par la méthode BET.
La composition peut comporter uniquement un ou plusieurs pigments composites tels que définis plus haut ou en variante comporter un ou plusieurs pigments composites autres ainsi que des pigments présentant une structure non composite, notamment des pigments minéraux, interférentiels, des laques ou des pigments organiques. La composition peut notamment être dépourvue de particules de $TiO_2$ non enrobées.

### *Noyau inorganique*

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.
De préférence, le rapport de la plus grande dimension du noyau à sa plus petite dimension est compris entre 1 et 50.
Le noyau inorganique peut présenter une taille moyenne comprise entre environ 5 nm et environ 100 nm, voire entre environ 5 nm et environ 75 nm, par exemple entre environ 10 nm et environ 50 nm, notamment autour de 20 ou 25 nm.
Par « taille moyenne », on désigne la dimension donnée par la distribution granulométrique statistique à la moitié de la population, dite D50. La taille moyenne peut être une taille moyenne en nombre déterminée par analyse

d'image (micro scopie électronique).

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment $TiO_2$, de fer, notamment $Fe_2O_3$, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 $m^2/g$ et environ 1000 $m^2/g$, mieux entre environ 10 $m^2/g$ et environ 600 $m^2/g$, par exemple entre environ 20 $m^2/g$ et environ 400 $m^2/g$, notamment entre 25 et 75 $m^2/g$, voire entre 40 et 60 $m^2/g$, notamment 50 $m^2/g$ environ.

La surface spécifique est par exemple comprise entre 30 et 70 $m^2/g$, par exemple voisine de 50 $m^2/g$, notamment dans le cas de noyaux de $TiO_2$ de taille voisine de 20 ou 25 nm.

Le noyau inorganique peut être coloré, le cas échéant.

L'indice de réfraction du noyau inorganique est avantageusement supérieur ou égal à 2, voir supérieur ou égal à 2,1 ou 2,2.

La proportion massique du noyau inorganique au sein du pigment composite peut excéder 50 %, étant par exemple comprise entre 50 et 70 %, ou 50 et 60 %.

***<u>Matière colorantes organique</u>***

La matière colorante organique peut être choisie par exemple parmi les composés particulaires insolubles dans le milieu physiologiquement acceptable de la composition.

La matière colorante organique peut comporter par exemple des pigments organiques qui peuvent être choisis parmi les composés ci-dessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels organiques insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

Dans certains exemples de réalisation, la teneur totale en matière organique colorante de la composition est inférieure ou égale à 10 % en masse par rapport au poids total de la composition.

La matière colorante organique représente par exemple entre 30 et 50 % en masse du pigment composite par rapport au poids total de celui-ci, par exemple entre 30 et 40%.

Avantageusement la composition est caractérisée par le fait que la matière colorante organique comporte au moins un pigment organique, la saturation $C^*_{masse}$ de la composition étant supérieure à 25.

***Liant***

Le liant peut être de tout type dès lors qu'il permet à la matière colorante organique d'adhérer à la surface du noyau inorganique. Le liant peut être organique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :

- les organosilanes (1) obtenus à partir d'alkoxysilanes,
- les polysiloxanes (2) modifiés ou non choisis parmi une liste non limitative comprenant :
- les polysiloxanes modifiés (2A) comprenant au moins un radical choisi parmi, notamment, les polyéthers, les polyesters et les composés époxy (ils seront appelés « polysiloxanes modifiés »),
- les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

$$R^1_a\ Si\ X_{4-a} \qquad (I)$$

dans laquelle :

- $R^1$ représente $C_6H_5$-, $(CH_3)_2$ $CH$-$CH_2$- ou un radical de type $C_b\ H_{2b+1}$- (où b varie de 1 à 18),
- X représente $CH_3O$- ou $C_2H_5O$-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) :

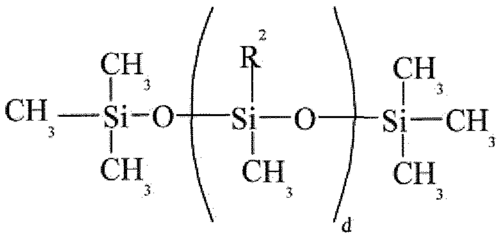

(II)

dans laquelle $R^2$ représente H- ou $CH_3$- et d varie de 15 à 450.

Parmi ces polysiloxanes, ceux pour lesquels $R^2$ représente H sont préférés.

Les polysiloxanes modifiés (2A) peuvent notamment répondre aux formules suivantes :

- ($a^1$) polysiloxanes modifiés portant des polyéthers, représentés par la formule (III)

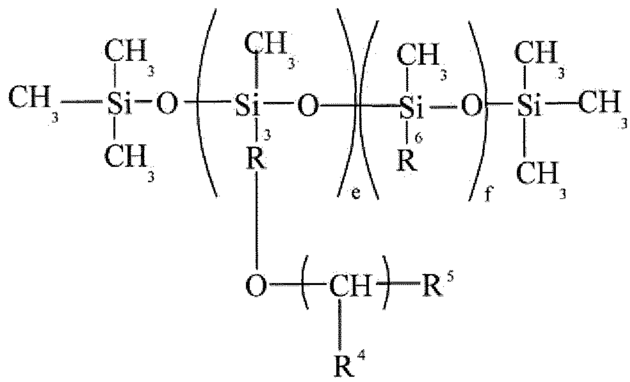

(III)

dans laquelle $R^3$ représente $-(CH_2)_h-$ ; $R^4$ représente $-(CH_2)_i- CH_3$ ; $R^5$ représente -OH, - COOH, -CH = $CH_2$, -C ($CH_3$) = $CH_2$ ou $-(CH_2)_j- CH_3$ ; $R^6$ représente -(CH2)$_k$- $CH_3$ ; g et h variant indépendamment de 1 à 15 ; j et k variant indépendamment de 0 à 15 ; e variant de 1 à 50 et f variant de 1 à 300,

- ($a^2$) polysiloxanes modifiés portant des polyesters, représentés par la formule (IV) :

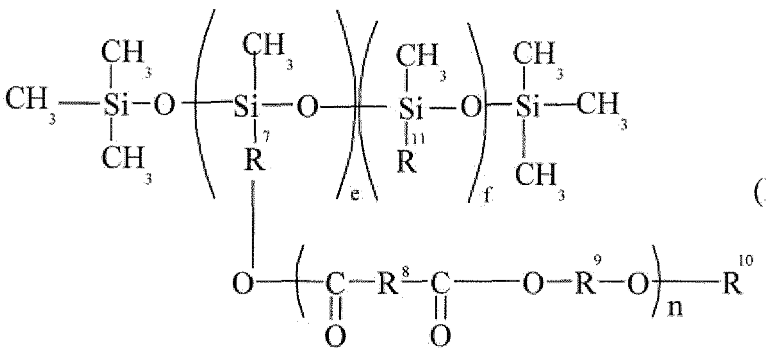

(IV)

dans laquelle $R^7$, $R^8$ et $R^9$ représentent indépendamment $-(CH_2)_q-$ ; $R^{10}$ représente -OH ; -COOH, -CH = $CH_2$, -C($CH_3$) = $CH_2$ ou $-(CH_2)_r- CH_3$ ; $R^{11}$ représente $-(CH_2)_s- CH_3$ ; n et q variant indépendamment de 1 à 15, r et s variant indépendamment de 0 à 15 ; e variant de 1 à 50 et f variant de 1 à 300,

- ($a^3$) polysiloxanes modifiés portant des radicaux époxy représentés par la formule (V):

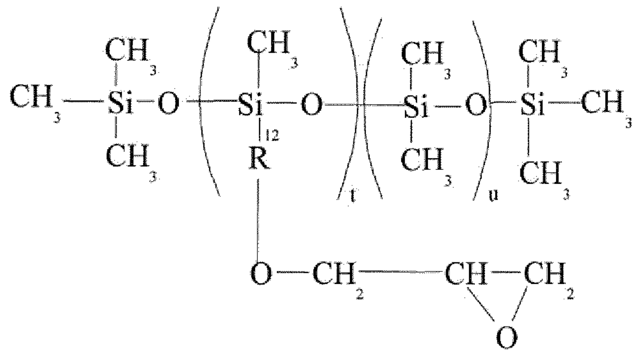

(V)

dans laquelle $R^{12}$ représente $-(CH_2)_v-$ ; v variant de 1 à 15 ; t variant de 1 à 50 et u variant de 1 à 300 ; ou leurs mélanges.

Parmi les polysiloxanes modifiés (2A), les polysiloxanes modifiés portant des polyéthers de formule (III) sont préférés.

Les polysiloxanes modifiés sur la partie terminale (2B) peuvent répondre à la formule (VI) :

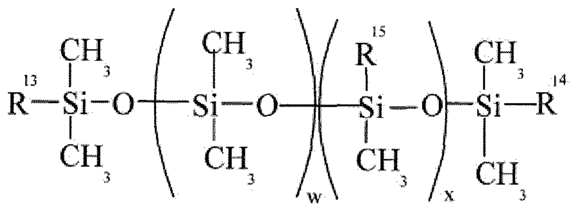

(VI)

dans laquelle $R^{13}$ et $R^{14}$ peuvent représenter -OH, $R^{16}$-OH ou $R^{17}$-COOH, indépendamment l'un de l'autre ; $R^{15}$ représente $-CH_3$ ou $-C_6H_5$ ; $R^{16}$ et $R^{17}$ représentent-$(CH_2)_y$- ; y variant de 1 à 15 ; w variant de 1 à 200 et x variant de 0 à 100.

Parmi ces polysiloxanes modifiés sur au moins une extrémité, ceux portant au moins radical ($R^{16}$ et/ou $R^{17}$) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

$$CF_3(CF_2)_zCH_2CH_2\ (R^{18})_aSiX_{4-a} \qquad (VII)$$

dans laquelle :

- $R^{18}$ représente $CH_3$-, $C_2H_5$-, $CH_3O$- ou $C_2H_5O$-,
- X représente $CH_3O$- ou $C_2H_5O$-,
- Z varie de 0 à 15 et a varie de 0 à 3.

Les fluoroalkylsilanes peuvent notamment être choisis dans une liste non limitative comprenant notamment le trifluoropropyltriméthoxysilane, le tri décafluorooctyltriméthoxysilane, l'heptadécafluorodécyltriméthoxysilane, l'heptadécafluorodécylméthyldiméthoxysilane, le trifluoropropyltriéthoxysilane, le e tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)-γ-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthylaminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium,

le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2.

La proportion relative massique de liant peut être inférieure ou égale à 5 %, voire à 3 %, par rapport au poids total du pigment composite.

***<u>Préparation du pigment composite</u>***

Le pigment composite peut être préparé partout procédé approprié, par exemple un procédé mécano-chimique ou un procédé de précipitation en solution, avec dissolution d'une substance colorante organique et précipitation à sa surface du noyau.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

***<u>AUTRES COMPOSANTS</u>***

***<u>Solvants</u>***

La composition peut comporter au moins un solvant aqueux ou organique.

Lorsque la composition comprend un ou plusieurs solvants organiques, ces solvants peuvent être présents en une teneur allant de 0,1 % à 99 %, par rapport au poids total de la composition.

D'une manière générale, la quantité de solvant(s), notamment organique(s), dépendra de la nature du support sur lequel la composition est destinée à être appliquée.

Dans le cas d'un vernis à ongles, par exemple, le solvant organique pourra être présent dans la composition à une teneur allant par exemple de 30 à 99 % en poids et de préférence de 60 % à 90 % en poids, par rapport au poids total de la composition.

La composition peut comporter au moins un solvant organique choisi dans la liste suivante :

- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La composition peut, en outre, contenir des éthers en $C_2$ et des aldéhydes en $C_2$-$C_4$ hydrophiles. L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

***Phase grass***

La composition, notamment lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes, et leurs mélanges. La phase grasse peut, en outre, contenir des solvants organiques lipophiles.

La composition peut présenter par exemple une phase grasse continue, pouvant contenir moins de 5% d'eau, notamment moins de 1% d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible,

ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

Notamment dans des exemples de réalisation où l'on cherche de la brillance, la composition peut comporter au moins une huile brillante choisie parmi les huiles non volatiles de masse moléculaire élevée.

La composition peut avantageusement contenir une huile non volatile de masse moléculaire élevée, par exemple comprise entre 650 à 10000 g/mol, notamment dans le cas d'une composition à appliquer sur les lièvres.

La composition peut contenir de 2 à 30 %, de préférence de 5 à 25 %, de 5 à 15 % d'au moins une huile de masse molaire allant de 650 à 10000 g/mol, et de préférence allant de 900 et 7500 g/mol.

L'huile non volatile de masse moléculaire élevée peut être une huile apolaire, par exemple une huile apolaire ayant une masse moléculaire comprise entre 300 et 900 g/mol.

L'huile de masse moléculaire allant de 650 à 10000 g/mol peut être choisie parmi :

- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
- les polydécènes et les polydécènes hydrogénés tels que le PURESYN 150 (MM=9200 g/mol) commercialisé par la société MOBIL CHEMICALS,
- les copolymères de la vinylpyrrolidone tels que le copolymère vinylpyrrolidone/1-héxadécène, ANTARON V-216 commercialisé ou fabriqué par la société ISP (MM=7300 g/mol),
- les esters tels que :

    a) les esters d'acides gras linéaires ayant un nombre total de carbone allant de 35 à 70 comme le tétrapélargonate de pentaérythrityle (MM=697,05 g/mol),
    b) les esters hydroxylés tels que le triisostéarate de polyglycérol-2 (MM=965,58 g/mol),
    c) les esters aromatiques tels que le tridécyl trimellitate (MM=757,19 g/mol),
    d) les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
    e) les esters et polyesters de dimère diol, tels que les esters de dimère diol et d'acide gras, et les esters de dimère diols et de diacide.

Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.

A titre illustratif des exemples d'acide mono-carboxylique, on peut notamment citer les acides gras.

Les esters de dimère diol et d'acide dicarboxylique peuvent être obtenus à partir d'un dimère diacide dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.

Selon une variante particulière, il s'agit plus particulièrement du dimère diacide dont dérive également le dimère diol à estérifier.

Les esters de dimère diol peuvent être obtenus à partir d'un dimère diol produit par hydrogénation catalytique d'un dimère diacide, par exemple le diacide dilinoléique hydrogéné.

A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7®.

- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

Dans des exemples de mise en oeuvre de l'invention, les huiles non volatiles peuvent représenter de 0,001 à 90 % du poids total de la composition, de préférence de 0,05 à 60 % et mieux de 1 à 35 %.

***Polymère filmogène***

La composition peut encore comporter, par exemple, un polymère filmogène, notamment dans le cas d'un mascara ou d'un vernis à ongles. "Polymère filmogène" désigne un[2] polymère apte à former à lui seul ou en présence d'un agent auxiliaire de filmification, un film continu et adhérent sur un support, notamment sur les matières kératiniques.

Parmi les polymères filmogènes utilisables dans une composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés $\alpha,\beta$-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEI KOGYO; ou encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER ; les sulfopolyesters vendus sous le nom de marque Eastman AQ par la société Eastman Chemical Products.

La composition selon l'invention peut comprendre également un agent auxiliaire de filmification favorisant la formation d'un film avec le polymère filmogène.

***Charges***

La composition peut comprendre en outre des charges. Par « charges », on désigne des particules de toute forme, insolubles dans le milieu de la composition, quelle que soit la température à laquelle la composition est fabriquée. Ces charges peuvent servir notamment à modifier la rhéologie ou la texture de la composition. La nature et la quantité des corps solides sont fonction des propriétés mécaniques et des textures recherchées.

A titre d'exemple de charges, on peut citer, entre autres, le talc, le mica, la silice, le kaolin, la séricite, les poudres de polyamide, de polyéthylène, de polytétrafluoroéthylène, de polyméthacrylate de méthyle, de polyuréthane, les poudres d'amidon et les billes de résine de silicone.

***Matière colorante additionnelle***

La composition peut comprendre une matière colorante additionnelle, différente du pigment composite utilisé dans la présente invention.
La matière colorante additionnelle peut être choisie parmi les pigments minéraux, les pigments organiques, les pigments nacrés, les colorants liposolubles ou hydrosolubles.
Les pigments minéraux peuvent être blancs ou colorés, enrobés ou on. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).
Parmi les pigments organiques, on peut citer le noir de carbone, les pigments de type D & C, et les laques à base de carmin de cochenille, de baryum, strontium, calcium, aluminium.
Les colorants liposolubles sont par exemple le rouge Soudan, le DC Red 17, le DC Green 6, le $\beta$-carotène, l'huile de soja, le brun Soudan, le DC Yellow 11, le DC Violet 2, le DC orange 5, le jaune quinoléine.
Les colorants hydrosolubles sont par exemple le jus de betterave et le bleu de méthylène.
Les colorants peuvent représenter de 0,1 à 20 % du poids de la composition et mieux de 0,1 à 6 % (si présents).
La quantité totale de matière colorante organique peut par exemple être inférieure à 10 % au sein de la composition, dans certains exemples de mise en oeuvre.

***Autres ingrédients***

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.
La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, les colorants ou leurs mélanges.
La composition selon l'invention peut comprendre, selon le type d'application envisagée, les constituants classiquement utilisés dans les domaines considérés, qui sont présents en une quantité appropriée à la forme galénique souhaitée.

***Formes galéniques***

La composition peut se présenter sous diverses formes, en fonction de sa destination. La composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau.
La composition peut se présenter sous forme de produit coulé, notamment de stick dans le cas d'un rouge à lèvres ou d'un produit de soin des lèvres.
La composition peut encore se présenter sous diverses autres formes, par exemple d'un liquide plus ou moins visqueux, d'un gel ou d'une pâte.
La composition peut encore se présenter sous la forme d'un solide, par exemple un pain à humidifier au moment de l'utilisation, de manière à lui permettre de se déliter.
La composition cosmétique peut constituer une composition de maquillage, entre autres, un rouge à lèvres,

un gloss liquide, une pâte de rouge à lèvres, un fard à joues, un crayon à lèvres, un fond de teint solide ou fluide, un produit anti-cernes ou de contour des yeux, un eye-liner, un mascara, un vernis à ongles, une ombre à paupières, un produit de maquillage du corps ou des cheveux ou encore un produit solaire ou de coloration de la peau.

L'invention a ainsi encore pour objet un rouge à lèvres, liquide ou solide, comportant une composition telle que définie plus haut.

L'invention a encore pour objet un fond de teint comportant une composition telle que définie plus haut.

L'invention a encore pour objet un vernis à ongles comportant une composition telle que définie plus haut.

L'invention a encore pour objet un mascara comportant une composition telle que définie plus haut.

L'invention a encore pour objet un produit de coloration des fibres capillaires comportant une composition telle que définie plus haut.

L'invention a encore pour objet un procédé de maquillage de la peau, des lèvres ou des phanères, dans lequel on applique sur la peau, les lèvres ou les phanères une composition telle que définie plus haut.

***EXEMPLES PROPOSES***

Les proportions sont massiques.

Les exemples 1 à 8 concernent plus particulièrement l'obtention de nouvelles teintes grâce à la sélection de pigments composites saturés et de $\Delta a^*b^*_{pigment}$ relativement élevé.

Les exemples 3, 4, 7, 9, 10, 11 et 12 concernent des compositions présentant une faible variation de la couleur avant et après application $\Delta E$.

Les exemples 1 à 3, 7, 8 et 11 concernent des compositions brillantes et saturées.

On a illustré à la figure 1 la variation des valeurs a* et b* pour les matières colorantes RED7 et RED28 LAKE pures et enrobant des pigments composites tels que définis sous * aux exemples 7 et 8. On a $\Delta a^*b^*_{pigment}$ supérieur à 5.

Exemple 1 : Vernis à ongles base solvant)

| | |
|---|---|
| Nitrocellulose | 19 % |
| N éthyl o,p toluenesulphonamide | 6 % |
| Citrate de Tributyl acétyle | 6 % |
| Hectorite | 1.2 % |
| Pigment composite* | 2 % |
| Isopropanol | 8 % |
| Acétate d'éthyle / acétate de butyle | ql 100 % |

* Mélange dioxide de titane synthétique**, D&C RED 7, polyméthylhydrogénosiloxane (proportions massiques respectives $T_iO_2$ : 65.8 / D&C RED 7 : 32.9 / liant : 1.3)

** $T_iO_2$ ayant une surface spécifique BET de 50 $m^2/g$ et une taille moyenne de 20 nm.

Exemple 2 : Vernis à ongles (base aqueuse)

| | |
|---|---|
| Latex (PU, 35 % matières solides) | 72.5 % |
| Agent gélifiant (Laponite XLS) | 1.2 % |
| Pigment composite * | 1 % |
| Eau | ql 100 % |

* Mélange dioxide de titane synthétique**, D&C RED 7, polyméthylhydrogénosiloxane (proportions massiques respectives $T_iO_2$ : 65.8 / D&C RED 7 32.9/liant : 1.3)

** $T_iO_2$ ayant une surface spécifique BET de 50 $m^2/g$ et une taille moyenne de 20 nm.

Exemple 3 : Rouge à lèvres

| | |
|---|---|
| Cire microcristalline | 2 % |
| Ozokerite | 5 % |
| Cire Candelilla | 7 % |
| Cire Carnauba | 3 % |
| Triglycérides d'acides capric/caprylic | 18 % |
| Octyldodécanol | 10 % |

(suite)

| | |
|---|---|
| Huile de lanoline | 6 % |
| Huile de lanoline acétylée | 6 % |
| Pigment composite* | 9 % |
| Parfum | 0.5 % |
| Huile de ricin | ql 100 % |

* Mélange dioxide de titane synthétique**, D&C RED 7, polyméthylhydrogénosiloxane (proportions massiques respectives $T_iO_2$ : 65.8 / D&C RED 7 : 32.9 / liant : 1.3)
** $T_iO_2$ ayant une surface spécifique BET de 50 $m^2/g$ et une taille moyenne de of 20 nm.

Exemple 4 : Fond de teint

Phase grasse

| | |
|---|---|
| - Surfactant commercialisé sous la marque commerciale "Abil WE 09" par la compagnie Goldschmidt | 8 % |
| - Cyclométhicone | 23 % |
| - Isododécane | 10 % |
| - $T_iO_2$ | 7 % |
| - Pigment composite* | 0.5 % |
| - Pigment d'oxyde de fer | 2.5 % |
| - Poudre de Nylon | 5 % |

Phase aqueuse

| | |
|---|---|
| - Eau déminéralisée | 42 % |
| - Sulfate de magnésium | 1 % |
| - Conservateurs | 1 % |

* Mélange dioxide de titane synthétique**, D&C RED 7, polyméthylhydrogénosiloxane (proportions massiques respectives $T_iO_2$ : 65.8 / D&C RED 7 : 32.9 / liant : 1.3)
** $T_iO_2$ ayant une surface spécifique BET de 50 $m^2/g$ et une taille moyenne de 20 nm.

Exemple 5: Mascara

| | |
|---|---|
| - Cire de paraffine | 2 % |
| - Cire de Carnauba | 4 % |
| - Cire d'abeilles | 8 % |
| - Polylvinyllaurate (Mexomer PP de Chimex) | 0.8 % |
| - Acétate de vinyle/ stéarate d'allyle copolymère (65/35) | 2 % |
| - Amidon de riz | 1 % |
| - Bentone | 4 % |
| - Carbonate de propylène | 2 % |
| - Pigment composite* | 4 % |
| - Conservateurs | qs |
| - Isododécane | qs 100 % |

* Mélange dioxide de titane synthétique**, FD&C Blue 1 Al Lake, polyméthylhydrogénosiloxane (proportions massiques respectives : $T_iO_2$: 58.1 / FD&C Blue 1 Lake 40.7 / liant 1.2)
** $T_iO_2$ ayant une surface spécifique BET de 50 $m^2/g$ et une taille moyenne de 20 nm.

Exemple 6 : Produit de coloration capillaire

| | |
|---|---|
| - Pigment composite* | 0.5 % |
| - Hydroxyléthylcellulose | 0.768 % |
| - Surfactant non ionique : Alkyle (50/50 C8/C10) polyglucoside en solution aqueuse à 60 % | 6 % |
| - Alcool benzylique | 8 % |
| - Polyéthylène glycol (8EO) | 12 % |
| - Solution aqueuse ammoniacale qs pH | 9 % |
| - Conservateurs | qs |
| - Eau déminéralisée | qs 100 % |

* Mélange dioxide de titane synthétique**, D&C RED 7, polyméthylhydrogénosiloxane (proportions massiques respectives $T_iO_2$ : 65.8 / D&C RED 7 : 32.9 / liant : 1.3)
* $T_iO_2$ ayant une surface spécifique BET de 50 $m^2/g$ et une taille moyenne de 20 nm.

Example 7 : Rouge à lèvres

| | |
|---|---|
| - Octyldodécanol | 15.61 % |
| - BHT | 0,06 % |
| - Lanolate d'isopropyle | 9,60 % |
| - Huile de lanoline acétylée | 9,60 % |
| - Phényl triméthicone | 4,26 % |
| - Diisostéarylmalate | 13,07 % |
| - Huile de lanoline | 9,60 % |
| - Trimellitate de tridécyle | 10,40 % |
| - Polyéthylène | 8,8 % |
| - Cire microcristalline | 4 % |
| - Glycérides de coco hydrogénée | 5 % |
| - Pigment composite * | 10,00 % |

* Mélange dioxide de titane synthétique**, D&C RED 7, polyméthylhydrogénosiloxane (proportions massiques respectives $T_iO_2$ : 65.8 / D&C RED 7 : 32.9 / liant : 1.3)
** $T_iO_2$ ayant une surface spécifique BET de 50 $m^2/g$ et une taille moyenne de 20 nm.

Example 8 : Rouge à lèvres

| | |
|---|---|
| - Octyldodécanol | 15.61 % |
| - BHT | 0,06 % |
| - Lanolate d'isopropyle | 9,60 % |
| - Huile de lanoline acétylée | 9.60 % |
| - Phényl triméthicone | 4,26 % |
| - Diisostéarylmalate | 13.07 % |
| - Huile de lanoline | 9,60 % |
| - Tridecyl trimellitate | 10.40 % |
| - Polyéthylène | 8,8 % |
| - Cire microcristalline | 4 % |
| - Glycérides de coco hydrogénée | 5 % |
| - Pigment composite * | 10.00 % |

* Mélange dioxide de titane synthétique**, D&C RED 28, polyméthylhydrogénosiloxane (proportions respectives massiques $T_iO_2$: 65.8 /D&C RED 7: 32.9/ liant : 1.3)
** $T_iO_2$ ayant une surface spécifique BET de 50 $m^2/g$ et une taille moyenne de 20 nm.

Exemple 9 : Rouge à lèvres

| | |
|---|---|
| - Octyldodécanol | 14.42 % |
| - BHT | 0.06 % |
| - Lanolate d'isopropyle | 8.87 % |
| - Huile de lanoline acétylée | 8.87 % |
| - Phényl triméthicone | 3.94 % |
| - Diisostéarylmalate | 12.07 % |
| - Huile de lanoline | 8.87 % |
| - Trimellitate de tridécyle | 9.60 % |
| - Cire de polyéthylène | 8.8 % |
| - Cire microcristalline | 4 % |
| - Glycérides de coco hydrogénée | 5 % |
| - Pigments composite * | 15.0 % |
| - FD&C Blue 1 Al Lake | 0.5 % |

* Mélange dioxide de titane synthétique**, D&C RED 7, polyméthylhydrogénosiloxane (proportions respectives massiques $T_iO_2$ 65.8 / D&C RED 7 : 32.9 / liant : 1.3)
** $T_iO_2$ ayant une surface spécifique BET de 50 $m^2/g$ et une taille moyenne de 20 nm.

Pour cette composition, les mesures suivantes peuvent être effectuées

| | |
|---|---|
| - $L^*_{masse}$ | 29,7 |
| - $a^*_{masse}$ | 31,2 |
| - $b^*_{masse}$ | 7 |
| - $L^*_{application}$ | 33,0 |
| - $a^*_{application}$ | 41,7 |
| - $b^*_{application}$ | 9,9 |
| - $C^*_{application}$ | 42,9 |
| - Pouvoir couvrant | 95,4 |
| - $\Delta E$ | 10,9 |

Exemple 10 : Rouge à lèvres

| | |
|---|---|
| - Octyldodécanol | 13.45 % |
| - BHT | 0.05 % |
| - Lanolate d'isopropyle | 8.27 % |
| - Huile de lanoline acétylée | 8.27 % |
| - Phényl triméthicone | 3.67 % |
| - Diisostéarylmalate | 11.26 % |
| - Huile de lanoline | 8.27 % |
| - Trimellitate de tridécyle | 8.96 % |
| - Cire de polyéthylène | 8.8 % |
| - Cire microcristalline | 4 % |
| - Glycérides de coco hydrogénés | 5 % |
| - Premier pigment composite* | 15.0 % |
| - Deuxième pigment composite*** | 5 % |

* Mélange dioxide de titane synthétique**, FD&C Yellow 5 Al Lake, polyméthylhydrogénesesiloxane (proportions massiques respectives $TiO_2$: 65.8/FD&C Yellow 5 Al Lake 32.9/liant 1.3).
** $T_iO_2$ ayant une surface spécifique BET de 50 $m^2/g$ et une taille moyenne de 20 nm.
*** Mélange dioxide de titane synthétique**, D&C Red 7, polyméthylhydrogénosiloxane (proportions massiques respectives : $T_iO_2$: 65.8/ D&C Red 7 32.9/liant 1.3).

Exemple 11 : Brillant à lèvres

| | |
|---|---|
| - Polyacryladipate-2 de bis-diglycéryle | 17,5 % |
| - Malate de diisostéraryle | 9,5 % |
| - Trimellitate de tridécyle | 10% |
| - Triglycéride d'acide C18-36 | 19% |
| - Silice diméthyl silylate | 8% |
| - Pigment composite* | 8% |
| - Nacre | 3% |
| - Polyisobutène | 12% |
| - Tétraisostéarate de pentaerythrityle | 13 % |
| - Parfum, conservateur | qs |

* Mélange dioxide de titane synthétique**, D&C RED 7, polyméthylhydrogénosiloxane (proportions respectives massiques $T_iO_2$ : 65.8 / D&C RED 7 : 32.9 / liant : 1.3)
** $T_iO_2$ ayant une surface spécifique BET de 50 $m^2/g$ et une taille moyenne de 20 nm.

Exemple 12 : Rouge à lèvres

| | |
|---|---|
| - Octyldodécanol | 14,55 % |
| - BHT | 0,06 % |
| - Lanolate d'isopropyle | 8,93 % |
| - Huile de lanoline acétylée | 8,93 % |
| - Phényl triméthicone | 3,96 % |
| - Diisostéarylmalate | 12,16 % |
| - Huile de lanoline | 8,93 % |
| - Trimellitate de tridécyle | 9,68 % |
| - Polyéthylène | 8,8 % |
| - Cire microcristalline | 4 % |
| - Glycérides de coco hydrogénés | 5 % |
| - Pigment composite* | 15 % |

* Mélange dioxide de titane synthétique **, D&C RED 7, polyméthylhydrogénosiloxane (proportions massiques respectives $T_iO_2$: 65.8/D&C RED 7:32.9/liant 1.3).
* $T_iO_2$ ayant une surface spécifique BET de 50 $m^2/g$ et une taille moyenne de 20 nm.

Pour cette composition, les mesures suivantes peuvent être effectuées

| | |
|---|---|
| - $L^*_{masse}$ | 33.9 |
| - $a^*_{masse}$ | 45.6 |
| - $b^*_{masse}$ | 15.3 |
| - $L^*_{application}$ | 34.2 |
| - $a^*_{application}$ | 52.5 |
| - $b^*_{application}$ | 17.6 |
| - $C^*_{application}$ | 55.4 |
| - Pouvoir couvrant | 85 |
| - $\Delta E$ | 7.2 |

Bien entendu, l'invention n'est pas limitée aux exemples qui viennent d'être donnés.

Dans toute la description, y compris les revendications, l'expression « comportant un » doit être comprise comme étant synonyme de « comportant au moins un », sauf si le contraire est spécifié.

Les intervalles donnés doivent être compris bornes incluses, sauf si le contraire est spécifié.

Dispositifs de conditionnement

La composition peut être conditionnée sous de multiples formes, avec un applicateur ou non, selon la forme galénique.

La composition, lorsqu'elle se présente sous la forme d'un stick, est par exemple conditionnée avec un mécanisme comportant une coupelle portant le stick et des moyens d'entraînement de la coupelle, ces moyens d'entraînement comportant par exemple deux pièces pouvant tourner l'une relativement à l'autre pour transformer une rotation relative en un déplacement axial de la coupelle.

La composition peut être conditionnée dans un dispositif de conditionnement, tel qu'un boîtier, récipient ou étui, étanche au moins avant la première utilisation. Ce dispositif de conditionnement peut être réalisé au moins partiellement avec des matières thermoplastiques ou en variante sans aucune matière thermoplastique. Le dispositif de conditionnement peut comporter une polyoléfine. Le dispositif de conditionnement peut également comporter au moins un élément métallique, par exemple une coupelle, une âme métallique torsadée, une charnière, une bague, un capot.

Lorsque la composition est destinée à l'application sur les lèvres, et se présente sous la forme d'un stick, l'extrémité du stick peut présenter une forme de biseau.

Lorsque la composition est destinée à être appliquée au moyen d'un applicateur, l'applicateur comporte par exemple une mousse, un embout floqué ou non, un feutre, une brosse, un peigne, un pinceau.

Lorsque présent, l'applicateur peut se loger de manière amovible sur le dispositif de conditionnement contenant la composition. En variante, l'applicateur peut être fixé à demeure sur le dispositif de conditionnement contenant la composition. Le dispositif de conditionnement peut comporter un piston ou tout autre moyen pour permettre l'alimentation de l'applicateur avec la composition.

Le dispositif de conditionnement peut comporter un organe de distribution tel qu'une pompe ou une valve, notamment lorsque la composition est liquide.

Lorsque présent, l'applicateur peut comporter une tige reliée à un organe de fermeture du dispositif de conditionnement, cet organe de fermeture pouvant également constituer un organe de préhension, le cas échéant.

Le dispositif de conditionnement contenant la composition peut être pourvu d'un fermoir ou autre moyen de fermeture, par exemple magnétique ou à encliquetage.

Le dispositif de conditionnement peut être pourvu d'un moyen de fermeture se fixant par vissage, friction ou encliquetage.

Le dispositif de conditionnement peut comporter des moyens d'étanchéité tels que par exemple une lèvre annulaire d'étanchéité ou un joint en élastomère, surinjecté ou rapporté.

Le dispositif de conditionnement contenant la composition peut comporter une étiquette ou une impression indiquant par exemple une marque ou un logo, cette impression ayant par exemple été réalisée par transfert à chaud ou à froid ou par sérigraphie ou par d'autres techniques d'impression.

Le dispositif de conditionnement contenant la composition peut comporter un emballage cartonné ou un blister, par exemple au moins partiellement réalisé en matière plastique transparente.

L'expression « comportant un » doit être comprise comme étant synonyme de « comportant au moins un », sauf si le contraire est spécifié.

## Revendications

1. Composition cosmétique comportant dans un milieu physiologiquement acceptable, de 0,1 à 85 % en poids d'huile par rapport à son poids total et au moins un pigment composite comportant un noyau inorganique comportant un matériau choisi parmi le dioxyde de titane et la silice et au moins un enrobage au moins partiel d'au moins une matière colorante organique, la taille moyenne du noyau inorganique étant comprise entre 5 nm et 100 nm, la variation $\Delta E$ de la couleur de la composition avant et après application étant inférieure ou égale à 20, mieux inférieure ou égale à 15, voire 10, ledit paramètre $\Delta E$ étant déterminé selon le protocole décrit dans la description et calculé selon :

$$\Delta E = [(a^*_{masse} - a^*_{application})^2 + (b^*_{masse} - b^*_{application})^2 + (L^*_{masse} - L^*_{application})^2]^{1/2}$$

dans laquelle $L^*_{masse}$, $a^*_{masse}$ et $b^*_{masse}$ désignent les valeurs L*, a* et b* de la composition dans la masse et $L^*_{application}$, $a^*_{application}$ et $b^*_{application}$ désignent les valeurs L*, a* et b* de la composition après application dans l'espace CIE L*a*b*.

2. Composition selon la revendication 1, **caractérisée par le fait que** le paramètre $\Delta_a{}^*b^*_{pigment}$ du pigment composite est supérieur ou égal à 5, mieux supérieur ou égal à 10, ou 15, mieux encore 20.

3. Composition selon la revendication 1 ou 2, **caractérisée par le fait que** la brillance moyenne $T_{oh}$ est supérieure ou égale à 30.

4. Composition selon l'une des revendications 1 à 3, **caractérisée par le fait qu'**elle comporte une phase grasse et que la teneur Q en particules de pigment composite de la composition est supérieure ou égale à 5 % relativement au poids total de la composition.

5. Composition selon l'une quelconque des revendications précédentes, **caractérisée par le fait que** la teneur en pigment composite dans la composition est comprise entre environ 0,1 % et environ 5 % en poids, par rapport au poids total de la composition, notamment entre environ 0,1 % et environ 3 % ou entre 0,5 % environ et 3 % environ.

6. Composition selon l'une quelconque des revendications précédentes, **caractérisée par le fait que** la saturation C* de la composition est comprise entre environ 30 et environ 100.

7. Composition selon l'une quelconque des revendications précédentes, **caractérisée par le fait que** la matière colorante organique comporte au moins un pigment organique, la saturation $C^*_{masse}$ de la composition étant supérieure à 25.

8. Composition selon l'une quelconque des revendications précédentes, **caractérisée par le fait que** la matière colorante organique comporte au moins une laque organique.

9. Composition selon l'une quelconque des revendications précédentes, **caractérisée par le fait que** le noyau inorganique présente une taille moyenne comprise entre environ 5 nm et environ 75 nm.

10. Composition selon la revendication précédente, **caractérisée par le fait que** le noyau inorganique présente une taille moyenne comprise entre 5 nm et 50 nm, bornes excluses.

11. Composition la revendication 10, **caractérisée par le fait que** le noyau inorganique présente une taille moyenne comprise entre environ 20 nm et environ 30 nm.

12. Composition selon l'une quelconque des revendications précédentes, **caractérisée par le fait que** le noyau inorganique présente une surface spécifique comprise entre environ 1 $m^2/g$ et environ 1000 $m^2/g$.

13. Composition selon la revendication précédente, **caractérisée par le fait que** la surface spécifique du noyau inorganique est comprise entre environ 10 $m^2/g$ et environ 600 $m^2/g$.

14. Composition selon la revendication12, **caractérisée par le fait que** la surface spécifique du noyau inorganique est comprise entre environ 20 $m^2/g$ et environ 400 $m^2/g$, notamment entre 25 et 75 $m^2/g$, voire entre 40 et 60 $m^2/g$, notamment 50 $m^2/g$ environ.

15. Composition selon l'une quelconque des revendications précédentes, **caractérisée par le fait que** le noyau inorganique présente une forme choisie parmi les suivantes : sphérique, globulaire, polyédrique, aciculaire, fusiforme, aplatie.

16. Composition selon l'une quelconque des revendications précédentes, **caractérisée par le fait que** la proportion massique de matière colorante organique est comprise entre environ 10 et environ 500 parts en poids pour 100 parts du noyau inorganique.

17. Composition selon la revendication précédente, **caractérisée par le fait que** la proportion massique de la matière colorante organique est comprise entre environ 20 et environ 250 parts en poids pour 100 parts du noyau inorganique.

18. Composition selon la revendication précédente, **caractérisée par le fait que** la proportion massique de la matière colorante organique est comprise entre environ 40 et environ 125 parts en poids pour 100 parts du noyau inorganique.

19. Composition selon l'une quelconque des revendications précédentes, **caractérisée par le fait que** la matière colorante organique est choisie parmi un carmin de cochenille, un pigment organique de colorant azoïque, anthraquinonique, indigoïde, xanthénique, pyrénique, quinolinique, de triphénylméthane, de fluorane, une laque organique, un sel insoluble de sodium, de potassium, de calcium, de baryum, d'aluminium, de zirconium, de strontium, de titane, de colorant acide.

**20.** Composition selon la revendication précédente, **caractérisée par le fait que** le colorant acide est choisi parmi un colorant azoïque, anthraquinonique, indigoïde, xanthénique, pyrénique, quinolinique, de triphénylméthane, de fluorane, et de tout autre colorant comportant au moins un groupe acide carboxylique ou sulfonique.

**21.** Composition selon l'une quelconque des revendications précédentes, **caractérisée par le fait que** la matière colorante organique comporte une laque organique qui est supportée par un support organique comportant au moins une colophane ou du benzoate d'aluminium.

**22.** Composition selon l'une quelconque des revendications 1 à 7, **caractérisée par le fait que** la matière colorante organique comporte un pigment organique ayant l'une des dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

**23.** Composition selon l'une quelconque des revendications 1 à 8 **caractérisée par le fait que** la matière colorante organique comporte une laque organique ayant l'une des dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

**24.** Composition selon l'une quelconque des revendications précédentes, **caractérisée par le fait que** le pigment composite comporte au moins un liant contribuant à la fixation de la matière colorante organique sur le noyau inorganique.

**25.** Composition selon la revendication précédente, **caractérisée par le fait que** le liant comporte au moins l'un d'un composé siliconé, d'un composé polymérique, et d'un composé oligomérique et similaire, comportant au moins l'un d'un organosilane, d'un organosilane fluoroalkylé, d'un polysiloxane, d'un agent couplant et d'un mélange de ceux-ci.

**26.** Composition selon la revendication 24 , **caractérisée par le fait que** le liant comporte du polyméthylhydrogénosiloxane.

**27.** Composition selon la revendication 24 , **caractérisée par le fait que** l'agent couplant est à base de silane, de titanate, d'aluminate et/ou de zirconate.

**28.** Composition selon la revendication 24, **caractérisée par le fait que** le liant comporte au moins un composé siliconé.

**29.** Composition selon l'une quelconque des revendications précédentes, **caractérisée par le fait que** le noyau inorganique est coloré.

30. Composition selon l'une quelconque des revendications précédentes, **caractérisée par le fait qu'**elle est dépourvue de particules de dioxyde de titane non enrobées.

31. Composition selon l'une quelconque des revendications précédentes, **caractérisée par le fait qu'**elle comporte au moins un actif cosmétique ou dermatologique.

32. Composition selon l'une quelconque des revendications précédentes, **caractérisée par le fait qu'**elle comporte au moins un corps gras, une cire, une gomme ou un polymère filmogène.

33. Composition selon l'une quelconque des revendications précédentes, **caractérisée par le fait qu'**elle comporte une matière colorante additionnelle différente du pigment composite.

34. Composition selon la revendication précédente, **caractérisée par le fait que** la matière colorante additionnelle est choisie parmi les pigments minéraux, les pigments organiques, les pigments nacrés, les colorants liposolubles et les colorants hydrosolubles.

35. Composition selon l'une quelconque des revendications précédentes, **caractérisée par le fait qu'**elle se présente sous une forme solide.

36. Composition selon l'une quelconque des revendications 1 à 34, **caractérisée par le fait qu'**elle se présente sous forme liquide, pâteuse ou gélifiée.

37. Composition selon la revendication 1, **caractérisée par le fait que** le pouvoir couvrant de la composition est compris strictement entre 1 et 25.

38. Composition selon la revendication 1, **caractérisée par le fait que** le pouvoir couvrant de la composition est compris strictement entre 25 et 100.

39. Composition selon l'une quelconque des revendications 1 à 36, **caractérisée en ce qu'**il s'agit d'un rouge à lèvres.

40. Composition selon l'une quelconque des revendications 1 à 36, **caractérisée en ce qu'**il s'agit d'un fond de teint.

41. Composition selon l'une quelconque des revendications 1 à 36, **caractérisée en ce qu'**il s'agit d'un vernis à ongles.

42. Composition selon l'une quelconque des revendications 1 à 36, **caractérisée en ce qu'**il s'agit d'un mascara.

43. Composition selon l'une quelconque des revendications 1 à 36, **caractérisée en ce qu'**il s'agit d'un produit de coloration des fibres capillaires.

44. Procédé de maquillage de la peau, des lèvres ou des phanères, dans lequel on applique sur la peau, les lèvres ou les phanères une composition telle que définie dans l'une quelconque des revendications précédentes.

45. Dispositif de conditionnement comportant une composition telle que définie dans l'une quelconque des revendications 1 à 38.

46. Dispositif selon la revendication 45, **caractérisé par le fait qu'**il est au moins partiellement réalisé avec au moins une matière thermoplastique.

47. Dispositif selon la revendication 45 ou 46, **caractérisé par le fait qu'**il comporte un applicateur.

48. Dispositif selon la revendication 45 ou 46, **caractérisé par le fait qu'**il est dépourvu d'applicateur.

## Patentansprüche

1. Kosmetische Zusammensetzung umfassend, in einem physiologisch verträglichen Medium, von 0,1 bis 85 Gew.-% Öl, bezogen auf ihr Gesamtgewicht, und mindestens ein Verbundpigment, das einen anorganischen Kern umfasst, der eine Substanz, ausgewählt aus Titan- und Siliciumdioxid, und mindestens einen Überzug mindestens teilweise

aus einem organischen Farbstoff umfasst, wobei die durchschnittliche Größe des anorganischen Kerns von 5 nm bis 100 nm beträgt, wobei die Schwankung ΔE der Farbe der Zusammensetzung vor und nach dem Aufbringen kleiner oder gleich 20, besser kleiner oder gleich 15; und sogar 10 beträgt; wobei der Parameter ΔE nach dem in der Beschreibung beschriebenen Protokoll bestimmt und gemäß:

$$\Delta E = [(a^*_{masse} - a^*_{application})^2 + (b^*_{masse} - b^*_{application})^2 + (L^*_{masse} - L^*_{application})^2]^{1/2}$$

berechnet wird, wobei $L^*_{masse}$, $a^*_{masse}$ und $b^*_{masse}$ die Werte L*, a* und b* der Zusammensetzung in der Masse bezeichnen und $L^*_{application}$, $a^*_{application}$ und $b^*_{application}$ die Werte L*, a* und b*der Zusammensetzung nach dem Aufbringen in dem Raum CIE L*a*b* bezeichnen.

2. Zusammensetzung nach Anspruch 1, **dadurch gekennzeichnet, dass** der Parameter $\Delta a^*b^*_{pigment}$ des Verbundpigments größer oder gleich 5, besser größer oder gleich 10, oder 15, noch besser 20 ist.

3. Zusammensetzung nach Anspruch 1 oder 2, **dadurch gekennzeichnet, dass** die durchschnittliche Leuchtkraft $T_{oh}$ größer oder gleich 30 ist.

4. Zusammensetzung nach einem der Ansprüche 1 bis 3, **dadurch gekennzeichnet, dass** sie eine Fettphase umfasst und dass der Gehalt Q an Verbundpigmentteilchen der Zusammensetzung größer oder gleich 5 % relativ zum Gesamtgewicht der Zusammensetzung beträgt.

5. Zusammensetzung nach einem der vorangehenden Ansprüche, **dadurch gekennzeichnet, dass** der Gehalt an Verbundpigment in der Zusammensetzung von etwa 0, 1 Gew.-% bis etwa 5 Gew.-%, bezogen auf das Gesamtgewicht der Zusammensetzung, insbesondere zwischen etwa 0,1 % bis etwa 3 % oder zwischen etwa 0,5 % und etwa 3% beträgt.

6. Zusammensetzung nach einem der vorangehenden Ansprüche, **dadurch gekennzeichnet, dass** die Farbsättigung C* der Zusammensetzung von etwa 30 bis etwa 100 beträgt.

7. Zusammensetzung nach einem der vorangehenden Ansprüche, **dadurch gekennzeichnet, dass** der organische Farbstoff mindestens ein organisches Pigment umfasst, wobei die Farbsättigung $C^*_{masse}$ der Zusammensetzung über 25 beträgt.

8. Zusammensetzung nach einem der vorangehenden Ansprüche, **dadurch gekennzeichnet, dass** der organische Farbstoff mindestens einen organischen Lack umfasst.

9. Zusammensetzung nach einem der vorangehenden Ansprüche, **dadurch gekennzeichnet, dass** der anorganische Kern eine durchschnittliche Größe von etwa 5 nm bis etwa 75 nm aufweist.

10. Zusammensetzung nach dem vorangehenden Anspruch, **dadurch gekennzeichnet, dass** der anorganische Kern eine durchschnittliche Größe von 5 nm bis 50 nm, Grenzen ausgeschlossen, aufweist.

11. Zusammensetzung nach Anspruch 10, **dadurch gekennzeichnet, dass** der anorganische Kern eine durchschnittliche Größe von etwa 20 nm bis etwa 30 nm aufweist.

12. Zusammensetzung nach einem der vorangehenden Ansprüche, **dadurch gekennzeichnet, dass** der anorganische Kern eine spezifische Oberfläche von etwa 1 $m^2/g$ bis etwa 1000 $m^2/g$ aufweist.

13. Zusammensetzung nach dem vorangehenden Anspruch, **dadurch gekennzeichnet, dass** die spezifische Oberfläche des anorganischen Kerns von 10 $m^2/g$ bis etwa 600 $m^2/g$ beträgt.

14. Zusammensetzung nach Anspruch 12, **dadurch gekennzeichnet, dass** die spezifische Oberfläche des anorganischen Kerns von etwa 20 $m^2/g$ bis etwa 400 $m^2/g$, insbesondere von 25 bis 75 $m^2/g$, und sogar von 40 bis 60 $m^2/g$, insbesondere etwa 50 $m^2/g$ beträgt.

15. Zusammensetzung nach einem der vorangehenden Ansprüche, **dadurch gekennzeichnet, dass** der anorganische

Kern eine aus den folgenden ausgewählte Form aufweist: sphärisch, kugelig, vielflächig, nadelförmig, spindelförmig, abgeflacht.

16. Zusammensetzung nach einem der vorangehenden Ansprüche, **dadurch gekennzeichnet, dass** der Massenanteil an organischem Farbstoff von etwa 10 bis etwa 500 Gewichtsteile auf 100 Teile des anorganischen Kerns beträgt.

17. Zusammensetzung nach dem vorangehenden Anspruch, **dadurch gekennzeichnet, dass** der Massenanteil des organischen Farbstoffs von etwa 20 bis etwa 250 Gewichtsteile auf 100 Teile des anorganischen Kerns beträgt.

18. Zusammensetzung nach dem vorangehenden Anspruch, **dadurch gekennzeichnet, dass** der Massenanteil des organischen Farbstoffs von etwa 40 bis etwa 125 Gewichtsteile auf 100 Teile des anorganischen Kerns beträgt.

19. Zusammensetzung nach einem der vorangehenden Ansprüche, **dadurch gekennzeichnet, dass** der organische Farbstoff ausgewählt ist aus Karminrot, einem organischen Pigment von Azofarbstoff, Anthrachinonfarbstoff, Indigofarbstoff, Xanthenfarbstoff, Pyrenfarbstoff, Chinolinfarbstoff, Triphenylmethanfarbstoff, Fluoranfarbstoff, einem organischen Lack, einem unlöslichen Salz von Natrium, Kalium, Calcium, Barium, Aluminium, Zirconium, Strontium, Titan; saurem Farbstoff.

20. Zusammensetzung nach dem vorangehenden Anspruch, **dadurch gekennzeichnet, dass** der saure Farbstoff ausgewählt ist aus einem Azofarbstoff, Anthrachinonfarbstoff, Indigofarbstoff, Xanthenfarbstoff, Pyrenfarbstoff, Chinolinfarbstoff, Triphenylmethanfarbstoff, Fluoranfarbstoff, und aus jedem anderen Farbstoff, der mindestens eine Carbonsäure- oder Sulfonsäuregruppe umfasst.

21. Zusammensetzung nach einem der vorangehenden Ansprüche, **dadurch gekennzeichnet, dass** der organische Farbstoff einen organischen Lack umfasst, der auf einem organischen Träger getragen wird, umfassend mindestens ein Kolophonium oder Aluminiumbenzoat.

22. Zusammensetzung nach einem der Ansprüche 1 bis 7, **dadurch gekennzeichnet, dass** der organische Farbstoff ein organisches Pigment mit einer der folgenden Bezeichnungen umfasst: D&C Blue no. 4, D&C Brown·n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, Red n° 28, D&C Red n° 30, D&GRed n° 31, D&C Red n° 33, D&C Red n° 34, D&C Red n° 36, D&C Violet n° 2, D&C Yellow n°. 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

23. Zusammensetzung nach einem der Ansprüche 1 bis 8, **dadurch gekennzeichnet, dass** der organische Farbstoff einen organischen Lack mit einer der folgenden Bezeichnungen umfasst: D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Alumimum/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

24. Zusammensetzung nach einem der vorangehenden Ansprüche, **dadurch gekennzeichnet, dass** das Verbundpigment mindestens ein Bindemittel umfasst, das zur Fixierung der organischen Substanz auf dem anorganischen Kern beiträgt.

25. Zusammensetzung nach dem vorangehenden Anspruch, **dadurch gekennzeichnet, dass** das Bindemittel mindestens eine von einer Silikonverbindung, einer Polymerverbindung und einer Oligomerverbindung und.ähnlich. umfasst, umfassend mindestens eines von einem Organosilan, einem Fluoralkylorganosilane, einem Polysiloxan, einem Kupplungsmittel und einem Gemisch davon.

**26.** Zusammensetzung nach Anspruch 24, **dadurch gekennzeichnet, dass** das Bindemittel Polymethylhydrogensiloxan umfasst.

**27.** Zusammensetzung nach Anspruch 24, **dadurch gekennzeichnet, dass** das Kupplungsmittel auf Silan-, Titanat-, Aluminat- und/oder Zirconatbasis ist.

**28.** Zusammensetzung nach Anspruch 24, **dadurch gekennzeichnet, dass** das Bindemittel mindestens eine Silikonverbindung umfasst.

**29.** Zusammensetzung nach einem der vorangehenden Ansprüche, **dadurch gekennzeichnet, dass** der anorganische Kern farbig ist.

**30.** Zusammensetzung nach einem der vorangehenden Ansprüche, **dadurch gekennzeichnet, dass** sie frei ist von nicht überzogenen Titandioxidteilchen.

**31.** Zusammensetzung nach einem der vorangehenden Ansprüche, **dadurch gekennzeichnet, dass** sie mindestens einen kosmetischen oder dermatologischen Wirkstoff umfasst.

**32.** Zusammensetzung nach einem der vorangehenden Ansprüche, **dadurch gekennzeichnet, dass** sie mindestens einen Fettkörper, ein Wachs, einen Gummi oder ein filmbildendes Polymer umfasst.

**33.** Zusammensetzung nach einem der vorangehenden Ansprüche, **dadurch gekennzeichnet, dass** sie einen zusätzlichen von dem Verbundpigment verschiedenen Farbstoff umfasst.

**34.** Zusammensetzung nach dem vorangehenden Anspruch, **dadurch gekennzeichnet, dass** der zusätzliche Farbstoff ausgewählt ist aus mineralischen Pigmenten, Lackpigmenten, fettlöslichen Farbstoffen und wasserlöslichen Farbstoffen.

**35.** Zusammensetzung nach einem der vorangehenden Ansprüche, **dadurch gekennzeichnet, dass** sie in Form eines Feststoffs vorliegt.

**36.** Zusammensetzung nach einem der Ansprüche 1 bis 34, **dadurch gekennzeichnet, dass** sie in flüssiger, pastöser oder gelartiger Form vorliegt.

**37.** Zusammensetzung nach Anspruch 1, **dadurch gekennzeichnet, dass** das Deckvermögen der Zusammensetzung streng von 1 bis 25 beträgt.

**38.** Zusammensetzung nach Anspruch 1, **dadurch gekennzeichnet, dass** das Deckvermögen der Zusammensetzung streng von 25 bis 100 beträgt.

**39.** Zusammensetzung nach einem der Ansprüche 1 bis 36, **dadurch gekennzeichnet, dass** es sich um einen Lippenstift handelt.

**40.** Zusammensetzung nach einem der Ansprüche 1 bis 36, **dadurch gekennzeichnet, dass** es sich um eine Teintgrundierung handelt.

**41.** Zusammensetzung nach einem der Ansprüche 1 bis 36, **dadurch gekennzeichnet, dass** es sich um einen Nagellack handelt.

**42.** Zusammensetzung nach einem der Ansprüche 1 bis 36, **dadurch gekennzeichnet, dass** es sich um einen Mascara handelt.

**43.** Zusammensetzung nach einem der Ansprüche 1 bis 36, **dadurch gekennzeichnet, dass** es sich um ein Haarfärbeprodukt handelt.

**44.** Schminkverfahren der Haut, Lippen oder der Phaneren, wobei auf die Haut, Lippen oder die Phaneren eine Zusammensetzung einem der vorangehenden Ansprüche aufgebracht wird.

**45.** Vorrichtung zum Aufbewahren umfassend eine Zusammensetzung nach einem der Ansprüche 1 bis 38.

**46.** Vorrichtung nach Anspruch 45, **dadurch gekennzeichnet, dass** sie mindestens zum Teil aus mindestens einem thermoplastischen Material realisiert ist.

**47.** Vorrichtung nach Anspruch 45 oder 46, **dadurch gekennzeichnet, dass** sie einen Applikator umfasst.

**48.** Vorrichtung nach Anspruch 45 oder 46, **dadurch gekennzeichnet, dass** sie ohne Applikator vorliegt.

## Claims

**1.** A cosmetic composition including in a physiologically acceptable medium, from 0.1 to 85% by weight of oil based on its total weight and at least one composite pigment including an inorganic core including a material selected from among titanium dioxide and silica and at least one at least partial coating of at least one organic coloring material, the average size of the inorganic core being comprised between 5 nm and 100 nm, the variation $\Delta E$ of the color of the composition before and after application being less than or equal to 20, better less than or equal to 15, or even 10, said parameter $\Delta E$ being determined according to the procedure described in the description and calculated according to:

$$\Delta E = [(a^*_{mass} - a^*_{application})^2 + (b^*_{mass} - b^*_{application})^2 + (L^*_{mass} - L^*_{application})^2)]^{½}$$

wherein $L^*_{mass}$, $a^*_{mass}$ and $b^*_{mass}$ refer to the values L*, a*, and b* of the composition in the bulk and $L^*_{application}$, $a^*_{application}$ and $b^*_{application}$ refer to the values L*, a*, and b* of the composition after application in the CIE space L*a*b*.

**2.** The composition according to claim 1, **characterized by** that the parameter $\Delta_{a^*b^*pigment}$ of the composite pigment is greater than or equal to 5, better greater than or equal to 10, or 15, still better 20.

**3.** The composition according to claim 1 or 2, **characterized by** the fact that the average brightness $T_{oh}$ is greater than or equal to 30.

**4.** The composition according to one of claims 1 to 3, **characterized by** the fact that it includes a fatty phase and that the content Q of composite pigment particles of the composition is greater than or equal to 5% relatively to the total weight of the composition.

**5.** The composition according to any of the preceding claims, **characterized by** the fact that the composite pigment content in the composition is comprised between about 0.1% and about 5% by weight, relatively to the total weight of the composition, notably between about 0.1% and about 3% or between about 0.5% and about 3%.

**6.** The composition according to any of the preceding claims, **characterized by** the fact that the saturation C* of the composition is comprised between about 30 and about 100.

**7.** The composition according to any of the preceding claims, **characterized by** the fact that the organic coloring material includes at least one organic pigment, the saturation $C^*_{mass}$ of the composition being greater than 25.

**8.** The composition according to any of the preceding claims, **characterized by** the fact that the organic coloring material includes at least one organic lacquer.

**9.** The composition according to any of the preceding claims, **characterized by** the fact that the inorganic core has an average size comprised between about 5 nm and about 75 nm.

**10.** The composition according to the preceding claim, **characterized by** the fact that the inorganic core has an average

size between 5 nm and 50 nm, limits excluded.

11. The composition according to claim 10, **characterized by** the fact that the inorganic core has an average size comprised between about 20 nm and about 30 nm.

12. The composition according to any of the preceding claims, **characterized by** the fact that the inorganic core has a specific surface area comprised between about 1 $m^2/g$ and about 1,000 $m^2/g$.

13. The composition according to the preceding claim, **characterized by** the fact that the specific surface area of the inorganic core is comprised between about 10 $m^2/g$ and about 600 $m^2/g$.

14. The composition according to claim 12, **characterized by** the fact that the specific surface area of the inorganic core is comprised between about 20 $m^2/g$ and about 400 $m^2/g$, notably between 25 and 75 $m^2/g$, or even between 40 and 60 $m^2/g$, notably about 50 $m^2/g$.

15. The composition according to any of the preceding claims, **characterized by** the fact that the inorganic core has a shape selected from the following shapes: spherical, globular, polyhedral, acicular, spindle-shaped, flattened.

16. The composition according to any of the preceding claims, **characterized by** the fact that the mass proportion of organic coloring material is comprised between about 10 and about 500 parts by weight for 100 parts of inorganic core.

17. The composition according to the preceding claim, **characterized by** the fact that the mass proportion of the organic coloring material is comprised between about 20 and about 250 parts by weight for 100 parts of the inorganic core.

18. The composition according to the preceding claim, **characterized by** the fact that the mass proportion of the organic coloring material is comprised between about 40 and about 125 parts by weight for 100 parts of the inorganic core.

19. The composition according to any of the preceding claims, **characterized by** the fact that the organic coloring material is selected from among cochineal carmine, an azoic, anthraquinone, indigoid, xanthenic, pyrenic, quinoline, triphenylmethane, fluorane coloring organic pigment, an organic lacquer, an insoluble sodium, potassium, calcium, barium, aluminium, zirconium, strontium, titanium salt of an acid coloring agent.

20. The composition according to the preceding claim, **characterized by** the fact that the acid coloring agent is selected from among an azoic, anthraquinone, indigoid, xanthenic, pyrenic, quinoline, triphenylmethane, fluorane coloring agent, or any other coloring agent including at least one carboxylic or sulfonic acid group.

21. The composition according to any of the preceding claims, **characterized by** the fact that the organic coloring material includes an organic lacquer which is supported by an organic support including at least one rosin or aluminium benzoate.

22. The composition according to any of claims 1 to 7, **characterized by** the fact that the organic coloring material includes an organic pigment having one of the following names: D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

23. The composition according to any of claims 1 to 8, **characterized by** the fact that the organic coloring material includes an organic lacquer having one of the following names: D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27

Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

**24.** The composition according to any of the preceding claims, **characterized by** the fact that the composite pigment includes at least one binder contributing to attachment of the organic coloring material onto the inorganic core.

**25.** The composition according to the preceding claim, **characterized by** the fact that the binder includes at least one of from a silicone compound, a polymeric compound, and an oligomeric compound and the like, including at least one from among an organosilane, a fluoroalkyl organosilane, a polysiloxane, a coupling agent and a mixture thereof.

**26.** The composition according to claim 24, **characterized by** the fact that the binder includes the polymethylhydrogenosiloxane.

**27.** The composition according to claim 24, **characterized by** the fact that the coupling agent is based on silane, titanate, aluminate and/or zirconate.

**28.** The composition according to claim 24, **characterized by** the fact that the binder includes at least one silicone compound.

**29.** The composition according to any of the preceding claims, **characterized by** the fact that the inorganic core is colored.

**30.** The composition according to any of the preceding claims, **characterized by** the fact that it is without any non-coated titanium dioxide particles.

**31.** The composition according to any of the preceding claims, **characterized by** the fact that it includes at least one cosmetic or dermatological active substance.

**32.** The composition according to any of the preceding claims, **characterized by** the fact that it includes at least one fat, one wax, one gum or film-forming polymer.

**33.** The composition according to any of the preceding claims, **characterized by** the fact that it includes an additional coloring material different from the composite pigment.

**34.** The composition according to the preceding claims, **characterized by** the fact that the additional coloring material is selected from among mineral pigments, organic pigments, nacreous pigments, fat-soluble coloring agents and water-soluble coloring agents.

**35.** The composition according to any of the preceding claims **characterized by** the fact that it appears in solid form.

**36.** The composition according to any of claims 1 to 34, **characterized by** the fact that it appears in liquid, pasty or gelled form.

**37.** The composition according to claim 1, **characterized by** the fact that the covering power of the composition is strictly comprised between 1 and 25.

**38.** The composition according to claim 1, **characterized by** the fact that the covering power of the composition is strictly comprised between 25 and 100.

**39.** The composition according to any of claims 1 to 36, **characterized in that** this is a lipstick.

**40.** The composition according to any of claims 1 to 36, **characterized in that** this is a foundation.

**41.** The composition according to any of claims 1 to 36, **characterized in that** this is a nail varnish.

**42.** The composition according to any of claims 1 to 36, **characterized in that** this is a mascara.

**43.** The composition according to any of claims 1 to 36, **characterized in that** this is a product for coloring capillary fibers.

**44.** A method for making up skin, lips or appendages, wherein a composition as defined in any of the preceding claims is applied on the skin, lips or appendages.

**45.** A conditioning device including a composition as defined in any of claims 1 to 38.

**46.** The device according to claim 45, **characterized by** the fact that it is at least partly made with at least one thermoplastic material.

**47.** The device according to claim 45 or 46, **characterized by** the fact that it includes an applicator.

**48.** The device according to claim a 45 or 46, **characterized by** the fact that it is without any applicator.

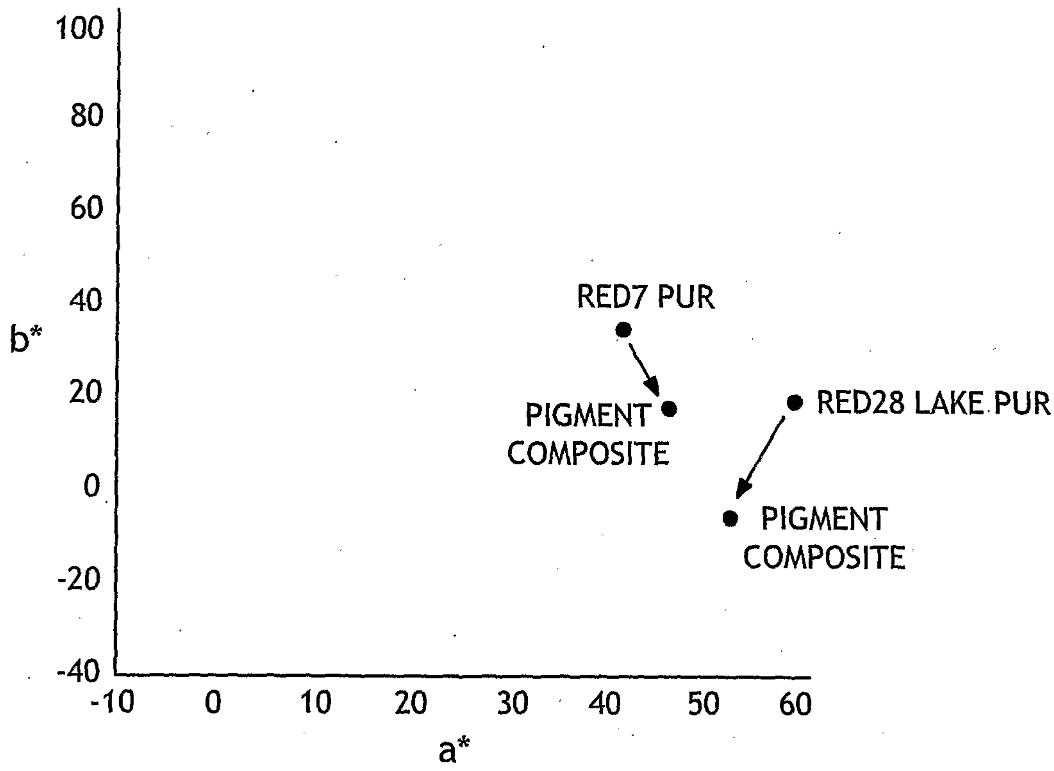
100
80
60
40
20
0
-20
-40
b*
-10
0
10
20
30
40
50
60
a*
RED7 PUR
PIGMENT
COMPOSITE
RED28 LAKE PUR
PIGMENT
COMPOSITE

FIG.1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5356617 A
- FR 2594130
- US 6428773 B
- EP 1217046 A
- EP 1184426 A
- EP 1184426 A2
- JP 3286463 B
- EP 0955039 A

### Littérature non-brevet citée dans la description

- International Cosmetic Ingredient Dictionnary and Handbook. The Cosmetic, Toiletry, and Fragrance Association, 1997, 371-386, 524-528